# EUROPEAN PATENT SPECIFICATION

(11) **EP 0 561 977 B1**
(45) Date of publication and mention of the grant of the patent: **26.05.2004**
(21) Application number: 92901997.4
(22) Date of filing: 12.12.1991
(51) Int. Cl.: A61K 31/41, A61K 31/415, A61P 9/10

(54) **USE OF ANGIOTENSIN II RECEPTOR ANTAGONISTS IN THE TREATMENT OF HEMORRAGIC STROKE**
VERWENDUNG VON ANGIOTENSIN-II-REZEPTORANTAGONISTEN IN DER BEHANDLUNG VON HÄMORRAGISCHEM SCHLAGANFALL
UTILISATION D'ANTAGONISTES DU RECEPTEUR DE L'ANGIOTENSINE II DANS LE TRAITEMENT D'ACCIDENTS HEMORRAGIQUES

(30) Priority: 14.12.1990 GB 9027199
(43) Date of publication of application: 29.09.1993
(73) Proprietor: SmithKline Beecham plc, Brentford, Middlesex TW8 9GS (GB)
(72) Inventor: HILL, James, Brentford, Middlesex TW8 9EP (GB)
(74) Representative: Thompson, Clive Beresford
(86) International application number: PCT/GB1991/002226
(87) International publication number: WO 1992/010188

(56) References cited:
- EP-A- 0 324 377
- EP-A- 0 403 158
- EP-A- 0 403 159

## Description

Use of Angiotensin II receptor antagonists in the treatment of haemorrhagic stroke.

### BACKGROUND OF THE INVENTION

Interruption of the renin-angiotensin system (RAS) with converting enzyme inhibitors, such as captopril, has proved clinically useful in the treatment of certain disease states, such as hypertension and congestive heart failure [Abrams, et al., Federation Proc., 43:1314 (1984)]. Furthermore, evidence suggests that inhibition of this system may be beneficial in the prevention of atheroma progression and the regression of atheroma. Since AII is the biologically active component of the renin-angiotensin system responsible for the system's peripheral effects, the most direct approach towards inhibition of RAS and in particular the prevention of atheroma progression and the regression of atheroma would be blockade of angiotensin II at its receptor.

### SUMMARY OF THE INVENTION

The present invention provides the use of an angiotensin II receptor antagonist in the manufacture of a medicament for the treatment of haemorrhagic stroke.

### DESCRIPTION OF THE INVENTION

The present invention relates to a therapeutic method for the treatment of haemorrhagic stroke in mammals. The method utilizes a class of antagonists which have been previously prepared and evaluated as effective AII receptor antagonists.

Substituted imidazoles of the formula (I) are described in U.S. Application Serial No. 07/746,262, filed August 14, 1991.

The present invention provides the use of an angiotensin II receptor antagonist of the formula (I): 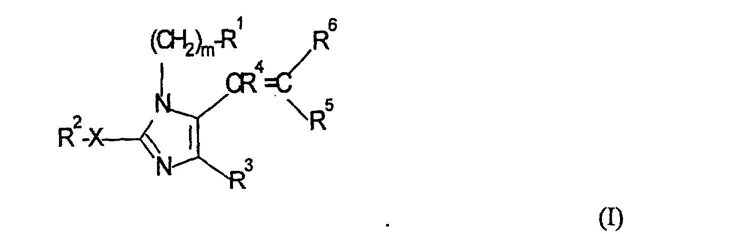 in which:
R¹ is adamantyl, phenyl, biphenyl, or naphthyl, with each aryl group being unsubstituted or substituted by one to three substituents selected from Cl, Br, F, I, C₁-C₆alkyl, nitro, A-CO₂R⁷, tetrazol-5-yl, C₁-C₆alkoxy, hydroxy, SC₁-C₆alkyl, SO₂NHR⁷, NHSO₂R⁷, SO₃H, CONR⁷R⁷, CN, SO₂C₁-C₆alkyl, PO(OR⁷)₂, NR⁷R⁷, NR⁷COH, NR⁷COC₁-C₆alkyl, NR⁷CON(R⁷)₂, NR⁷COW, W, SO₂W;
m is 0-4;
R² is C₂-C₁₀alkyl, C₃-C₁₀alkenyl, C₃-C₁₀alkynyl, C₃-C₆cycloalkyl, or (CH₂)₀₋₈phenyl unsubstituted or substituted by one to three substituents selected from C₁-C₆alkyl, nitro, Cl, Br, F, I, hydroxy, C₁-C₆alkoxy, NR⁷R⁷, CO₂R⁷, CN, CONR⁷R⁷, W, tetrazol-5-yl, NR⁷COC₁-C₆alkyl, NR⁷COW, SC₁-C₆alkyl, SO₂W, or SO₂C₁-C₆alkyl;
X is a single bond, S, NR⁷, or O;
R³ is hydrogen, Cl, Br, F, I, CHO, hydroxymethyl, COOR⁷, CONR⁷R⁷, NO₂, W, CN, NR⁷R⁷, or phenyl;
R⁴ and R⁵ are independently hydrogen, C₁-C₆alkyl, thienyl-Y-, furyl-Y-, pyrazolyl-Y-, imidazolyl-Y-, pyrrolyl-Y-, triazolyl-Y-, oxazolyl-Y-, isoxazolyl-Y-, thiazolyl-Y-, pyridyl-Y-, or tetrazolyl-Y-, except that R⁴ and R⁵ are not both selected from hydrogen and C₁-C₆alkyl and each heterocyclic ring is unsubstituted or substituted by C₁-C₆alkyl, C₁-C₆alkoxy, Cl, Br, F, I, NR⁷R⁷, CO₂R⁷, SO₂NHR⁷, SO₃H, or CONR⁷R⁷, OH, NO₂, W, SO₂W, SC₁-C₆alkyl, SO₂C₁-C₆alkyl, NR⁷COH, NR⁷COW, or NR⁷COC₁-C₆alkyl;
Y is a single bond, O, S, or C₁-C₆alkyl which is straight or branched or optionally substituted by phenyl or benzyl, wherein each of the aryl groups is unsubstituted or substituted by halo, NO₂, CF₃, C₁-C₆alkyl, C₁-C₆alkoxy, CN, or
CO₂R⁷; R⁶ is -Z-COOR⁸ or -Z-CONR⁷R⁷;
Z is a single bond, vinyl, -CH₂-O-CH₂-, methylene optionally substituted by C₁-C₆alkyl, one or two benzyl groups, thienylmethyl, or furylmethyl, or -C(O)NHCHR⁹-, wherein R⁹ is H, C₁-C₆alkyl, phenyl, benzyl, thienylmethyl, or furylmethyl;
W is CₙF₂ₙ₊₁, wherein n is 1-3;
A is -(CH₂)ₘ-, -CH=CH-, -O(CH₂)ₙ-, or -S(CH₂)ₙ-;
each R⁷ independently is hydrogen, C₁-C₆alkyl, or (CH₂)ₘphenyl, wherein m is 0-4; and
R⁸ is hydrogen, C₁-C₆alkyl, or 2-di(C₁-C₆alkyl)-amino-2-oxoethyl; or a pharmaceutically acceptable salt thereof;
in the manufacture of a medicament for the treatment of haemorrhagic stroke.

Preferred compounds included within the scope of formula (I) are:
(E)-3-[2-n-butyl-1-{(4-carboxyphenyl)methyl}-1H-imidazol-5-yl]-2-(2-thienyl)methyl-2-propenoic acid,
(E)-3-[2-n-butyl-1-{(4-carboxynaphth-1-yl)methyl}-1H-imidazol-5-yl]-2-(2-thienyl)methyl-2-propenoic acid,
(E)-3-[2-n-butyl-1-{(2-chloro-4-carboxyphenyl)-methyl}-1H-imidazol-5-yl]-2-(2-thienyl)methyl-2-propenoic acid, and
(E)-3-[2-n-butyl-1-{(4-carboxy-2,3-dichlorophenyl)-methyl}-1H-imidazol-5-yl]-2-(2-thienyl)methyl-2-propenoic acid; or a pharmaceutically acceptable salt thereof.

Particularly preferred compounds are (E)-3-[2-n-butyl-1-{(4-carboxyphenyl)methyl}-1H-imidazol-5-yl]-2-(2-thienyl)methyl-2-propenoic acid and (E)-3-[2-n-butyl-1-{(4-carboxynaphth-1-yl)methyl}-1H-imidazol-5-yl]-2-(2-thienyl)methyl-2-propenoic acid; or a pharmaceutically acceptable salt thereof.

The most preferred compound of this invention is (E)-3-[2-n-butyl-1-{(4-carboxyphenyl)methyl]-1H-imidazolyl-5-yl]-2-(2-thienyl)methyl-2-propenoic acid methanesulfonate.

The compounds of formula (I) are prepared following the methods described in European Patent Publication Number EP 0 403 159, published on December 19, 1990.

Other AII receptor antagonists for use in the present invention are disclosed in the following:
Carini et al., in European Patent Publication No. 253 310, published January 20, 1988 and U.S. Application Serial No. 50341 filed May 22, 1987, discloses 2-n-butyl-4-chloro-1-[(2'-(1H-tetrazol-5-yl)biphenyl-4-yl)methyl]-5-(hydroxymethyl)imidazole and pharmaceutically acceptable salts thereof.
Chakravarty, et al., European Patent Publication Number EP 0 400 974, published December 5, 1990 and U.S. Application Serial No. 07/516,286, filed May 4, 1990, discloses 5,7-dimethyl-2-ethyl-3-(2'-(tetrazol-5-yl)biphenyl-4-yl)methyl-3H-imidazo[4,5-b]pyridine and pharmaceutically acceptable salts thereof.
Carini, et al., European Patent Publication Number EP 0 324 377, published July 19, 1989 and U.S. Application Serial No. 07/279,194, filed December 6, 1988, discloses 2-n-propyl-4-pentafluoroethyl-1-[2'-(1H-tetrazol-5-yl)biphenyl-4-yl)methyl]imidazole-5-carboxylic acid and pharmaceutically acceptable salts thereof.
Roberts, et al., European Patent Publication Number EP 0 412 848, published February 13, 1991, discloses 2-methyl-4-(2'-(1H-tetrazol-5-yl)biphenyl-4-yl)methoxy]quinoline and pharmaceutically acceptable salts thereof.

The above descriptions of AII antagonists for use in the present invention were taken from pending patent applications, noted patents, and publications or from abstracts thereof. Reference should be made to such patents and publications themselves for their full disclosures of such compounds.

Certain AII antagonists employed in the invention may exist in isomeric form. This invention includes all such isomers both in pure form and admixture, including racemic mixtures and their pharmaceutically acceptable salts.

Angiotensin II antagonist activity is assessed by in vitro methods. In vitro antagonist activity is determined by the ability of the compounds to compete with ¹²⁵I-angiotensin II for binding to vascular angiotensin II receptors and by their ability to antagonize the contractile response to angiotensin II in the isolated rabbit aorta. For the purposes of the present invention the preferred AII antagonists are compounds which are capable of inhibiting the action of AII by at least 50% at a concentration of 1mM or less, and especially preferred AII antagonists are compounds which are capable of inhibiting the action of AII by at least 50% at a concentration of 25nM or less when tested by the following standard methods.

### Binding

The radioligand binding assay is a modification of a method previously described in detail (Gunther et al., Circ. Res. 47:278, 1980). A particular fraction from rat mesenteric arteries is incubated in Tris buffer with 80 pM of ¹²⁵I-angiotensin II with or without angiotensin II antagonists for 1 hour at 25°C. The incubation is terminated by rapid filtration and receptor bound ¹²⁵I-angiotensin II trapped on the filter is quantitated with a gamma counter. The potency of angiotensin II antagonists is expressed as the IC₅₀ which is the concentration of antagonist needed to displace 50% of the total specifically bound angiotensin II.

### Aorta

The ability of the compounds to antagonize angiotensin II induced vasoconstriction is examined in the rabbit aorta. Ring segments are cut from the rabbit thoracic aorta and suspended in organ baths containing physiological salt solution. The ring segments are mounted over metal supports and attached to force displacement transducers which are connected to a recorder. Cumulative concentration response curves to angiotensin II are performed in the absence of antagonist or following a 30-minute incubation with antagonist. Antagonist dissociation constants (K_{B}) are calculated by the dose ratio method using the mean effective concentrations.

In the therapeutic use for the prevention of atheroma progression and the regression of atheroma the AII receptor antagonizing compounds of this invention are incorporated into standard pharmaceutical compositions. They can be administered orally, parenterally, rectally, topically or transdermally.

The compounds and their pharmaceutically acceptable salts which are active when given orally can be formulated as liquids, for example syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example, polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

The compounds and their pharmaceutically acceptable salts which are active when administered parenterally (i.e. by injection or infusion) can be formulated as solutions or suspensions.

A composition for parenteral administration will generally consist of a solution or suspension of the active ingredient in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository composition comprises a compound or a pharmaceptically acceptable salt thereof which is active when administered in this way, with a binding and/or lubricating agent such as polymeric glycols, gelatins or coca butter or other low melting vegetable or synthetic waxes or fats.

A typical transdermal formulation comprises a conventional aqueous or non-aqueous vehicle, for example, a cream, ointment lotion or paste or in the form of a medicated plaster, patch or membrane.

For topical administration, the pharmaceutical compositions adapted include solutions, suspensions, ointments, and solid inserts. Typical pharmaceutically acceptable carriers are, for example, water, mixtures of water and water-miscible solvents such as lower alkanols or vegetable oils, and water soluble ophthalmologically acceptable non-toxic polymers, for example, cellulose derivatives such as methyl cellulose. The pharmaceutical preparation may also contain non-toxic auxiliary substances such as emulsifying, preserving, wetting, and bodying agents, as for example, polyethylene glycols; antibacterial components such as quaternary ammonium compounds; buffering ingredients such as alkali metal chloride; antioxidants such as sodium metabisulfite; and other conventional ingredients such as sorbitan monolaurate.

Preferably the composition is in unit dose form. Doses of the compounds in a pharmaceutical dosage unit will be an efficacious, non-toxic quantity selected from the range of 0.01 - 200 mg/kg of active compound, preferably 0.1 - 100 mg/kg. The selected dose is administered to a human patient in need of prevention of atheroma progression and the regression of atheroma induced by angiotensin II from 1-6 times daily, orally, rectally, topically, by injection, or continuously by infusion, Oral dosage units for human administration preferably contain from 10 to 500 mg of active compound. Lower dosages are used generally for parenteral administration. Oral administration is used when safe, effective, and convenient for the patient.

No unacceptable toxicological effects are expected when compounds are administered in accordance with the present invention.

The following examples are intended to illustrate, but not to limit, the present invention. The examples are directed to pharmaceutical compositions. The compounds included in these disclosed compositions are representative of the AII receptor antagonists included within the scope of the instant invention, but therapeutically effective amounts of other AII antagonists as discussed hereinabove may be substituted.

### Example 1

An oral dosage form for administering orally active Formula (I) compounds is produced by screening, mixing and filling into hard gelatin capsules the ingredients in proportions, for example, as shown below.

| Ingredients | Amounts |
|---|---|
| (E)-3-[2-n-butyl-1-{(4-carboxy-phenyl)methyl}-1H-imidazol-5-yl]-2-(2-thienyl)methyl-2-propenoic acid | 100 mg |
| magnesium stearate | 10 mg |
| lactose | 100 mg |

### Example 2

The sucrose calcium sulfate dihydrate and orally active Formula (I) compounds are mixed and granulated with a 10% gelatin solution. The wet granules are screened, dried, mixed with the starch, talc and stearic acid, screened and compressed into a tablet.

| Ingredients | Amounts |
|---|---|
| (E)-3-[2-n-propyl-1-{(4-carboxynaphth-1-yl)methyl}-1H-imidazol-5-yl]-2-(2-thienyl)methyl-2-propenoic acid | 75 mg |
| calcium sulfate dihydrate | 100 mg |
| sucrose | 15 mg |
| starch | 8 mg |
| talc | 4 mg |
| stearic acid | 2 mg |

### Example 3

(E)-3-[2-n-Butyl-1-{(4-carboxyphenyl)methyl}-1H-imidazol-5-yl]-2-(2-thienyl)methyl-2-propenoic acid, 50 mg, is dispersed in 25 ml of normal saline to prepare an injectable preparation.

It is to be understood that the invention is not limited to the embodiments illustrated hereabove and the right to the illustrated embodiments and all modifications coming within the scope of the following claims is reserved.

## Claims

1. Use of an angiotensin II receptor antagonist of the formula: 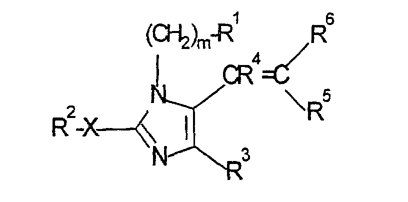 in which:
R¹ is adamantyl, phenyl, biphenyl, or naphthyl, with each aryl group being unsubstituted or substituted by one to three substituents selected from Cl, Br, F, I, C₁-C₆alkyl, nitro, A-CO₂R⁷, tetrazol-5-yl, C₁-C₆alkoxy, hydroxy, SC₁-C₆alkyl, SO₂NHR⁷, NHSO₂R⁷, SO₃H, CONR⁷R⁷, CN, SO₂C₁-C₆alkyl, PO(OR⁷)₂, NR⁷R⁷, NR⁷COH, NR⁷COC₁-C₆alkyl, NR⁷CON(R⁷)₂, NR⁷COW, W, SO₂W;
m is 0-4;
R² is C₂-C₁₀alkyl, C₃-C₁₀alkenyl, C₃-C₁₀alkynyl, C₃-C₆cycloalkyl, or (CH₂)₀₋₈phenyl unsubstituted or substituted by one to three substituents selected from C₁-C₆alkyl, nitro, Cl, Br, F, I, hydroxy, C₁-C₆alkoxy, NR⁷R⁷, CO₂R⁷, CN, CONR⁷R⁷, W, tetrazol-5-yl, NR⁷COC₁-C₆alkyl, NR⁷COW, SC₁-C₆alkyl, SO₂W, or SO₂C₁-C₆alkyl;
X is a single bond, S, NR⁷, or O;
R³ is hydrogen, Cl, Br, F, I, CHO, hydroxymethyl, COOR⁷, CONR⁷R⁷, NO₂, W, CN, NR⁷R⁷, or phenyl;
R⁴ and R⁵ are independently hydrogen, C₁-C₆alkyl, thienyl-Y-, furyl-Y-, pyrazolyl-Y-, imidazolyl-Y-, pyrrolyl-Y-, triazolyl-Y-, oxazolyl-Y-, isoxazolyl-Y-, thiazolyl-Y-, pyridyl-Y-, or tetrazolyl-Y-, except that R⁴ and R⁵ are not both selected from hydrogen and C₁-C₆alkyl and each heterocyclic ring is unsubstituted or substituted by C₁-C₆alkyl, C₁-C₆alkoxy, Cl, Br, F, I, NR⁷R⁷, CO₂R⁷, SO₂NHR⁷, SO₃H, or CONR⁷R⁷, OH, NO₂, W, SO₂W, SC₁-C₆alkyl, SO₂C₁-C₆alkyl, NR⁷COH, NR⁷COW, or NR⁷COC₁-C₆alkyl;
Y is a single bond, O, S, or C₁-C₆alkyl which is straight or branched or optionally substituted by phenyl or benzyl, wherein each of the aryl groups is unsubstituted or substituted by halo, NO₂, CF₃, C₁-C₆alkyl, C₁-C₆alkoxy, CN, or CO₂R⁷;
R⁶ is -Z-COOR⁸ or -Z-CONR⁷R⁷;
Z is a single bond, vinyl, -CH₂-O-CH₂-, methylene optionally substituted by C₁-C₆alkyl, one or two benzyl groups, thienylmethyl, or furylmethyl, or -C(O)NHCHR⁹-, wherein R⁹ is H, C₁-C₆alkyl, phenyl, benzyl, thienylmethyl, or furylmethyl;
W is CₙF₂ₙ₊₁, wherein n is 1-3;
A is -(CH₂)ₘ-, -CH=CH-, -O(CH₂)ₙ-, or -S(CH₂)ₙ-;
each R⁷ independently is hydrogen, C₁-C₆alkyl, or (CH₂)ₘphenyl, wherein m is 0-4; and
R ⁸ is hydrogen, C₁-C₆alkyl, or 2-di(C₁-C₆alkyl)-amino-2-oxoethyl; or a pharmaceutically acceptable salt thereof;
in the manufacture of a medicament for the treatment of haemorrhagic stroke.

2. Use of an angiotensin II receptor antagonist according to claim 1 which is (E)-3-[2-n-butyl-1-{(4-carboxyphenyl)methyl}-1H-imidazol-5-yl]-2-(2-thienyl)methyl-2-propenoic acid or a pharmaceutically acceptable salt thereof.

3. Use of an angiotensin II receptor antagonist according to claim 1 which is (E)-3-[2-n-butyl-1-{(4-carboxyphenyl)methyl]-1H-imidazolyl-5-yl]-2-(2-thienyl)methyl-2-propenoic acid methanesulfonate.

4. Use of an angiotensin II receptor antagonist according to claim 1 which is (E)-3-[2-n-butyl-1-{(4-carboxynaphth-1-yl)methyl}-1H-imidazol-5-yl]-2-(2-thienyl)methyl-2-propenoic acid or a pharmaceutically acceptable salt thereof.

5. Use of an angiotensin II receptor antagonist which is 2-n-butyl-4-chloro-1-[(2'-(1H-tetrazol-5-yl)biphenyl-4-yl)methyl]-5-(hydroxymethyl)-imidazole or a pharmaceutically acceptable salt thereof; in the manufacture of a medicament for the treatment of haemorrhagic stroke.

6. Use of an angiotensin II receptor antagonist which is 2-n-propyl-4-pentafluoroethyl-1-[(2'-(1H-tetrazol-5-yl)biphenyl-4-yl)methyl]imidazole-5-carboxylic acid or a pharmaceutically acceptable salt thereof; in the manufacture of a medicament for the treatment of haemorrhagic stroke.

7. Use of an angiotensin II receptor antagonist which is 5,7-dimethyl-2-ethyl-3-(2'-(tetrazol-5-yl)biphenyl-4-yl)methyl-3H-imidazo[4,5-b]pyridine or a pharmaceutically acceptable salt thereof; in the manufacture of a medicament for the treatment of haemorrhagic stroke.

8. Use of an angiotensin II receptor antagonist which is 2-methyl-4-[(2'-(1H-tetrazol-5-yl)biphenyl-4-yl)methoxy]quinoline or a pharmaceutically acceptable salt thereof; in the manufacture of a medicament for the treatment of haemorrhagic stroke.

## Patentansprüche

1. Verwendung eines Angiotensin-II-Rezeptorantagonisten der Formel: 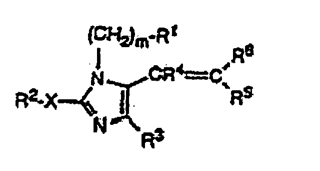 wobei:
R¹ Adamantyl, Phenyl, Biphenyl oder Naphthyl darstellt, wobei jede Arylgruppe unsubstituiert oder durch einen bis drei Substituenten, ausgewählt aus Cl, Br, F, I, C₁-C₆-Alkyl, Nitro, A-CO₂R⁷, Tetrazol-5-yl, C₁-C₆-Alkoxy, Hydröxy, SC₁-C₆-Alkyl, SO₂NHR⁷, NHSO₂R⁷, SO₃H, CONR⁷R⁷, CN, SO₂C₁-C₆-Alkyl, PO(OR⁷)₂, NR⁷R⁷, NR⁷COH, NR⁷COC₁-C₆-Alkyl, NR⁷CON(R⁷)₂, NR⁷COW, W, SO₂W substituiert ist;
m 0 bis 4 ist;
R² C₂-C₁₀-Alkyl, C₃-C₁₀-Alkenyl, C₃-C₁₀-Alkinyl, C₃-C₆-Cycloalkyl oder (CH₂)₀₋₈-Phenyl darstellt, welche unsubstituiert oder durch einen bis drei Substituenten ausgewählt aus C₁-C₆-Alkyl, Nitro, Cl, Br, F, I, Hydroxy, C₁-C₆-Alkoxy, NR⁷R⁷, CO₂R⁷, CN, CONR⁷R⁷, W, Tetrazol-5-yl, NR⁷COC₁-C₆-Alkyl, NR⁷COW, SC₁-C₆-Alkyl, SO₂W oder SO₂C₁-C₆-Alkyl substituiert sind;
X eine Einfachbindung, S, NR⁷ oder O darstellt;
R³ ein Wasserstoffatom, Cl, Br, F, I, CHO, Hydroxymethyl, COOR⁷, CONR⁷R⁷, NO₂, W, CN, NR⁷R⁷ oder Phenyl darstellt;
R⁴ und R⁵ unabhängig ein Wasserstoffatom, C₁-C₆-Alkyl, Thienyl-Y-, Furyl-Y-, Pyrazolyl-Y-, Imidazolyl-Y-, Pyrrolyl-Y-, Triazolyl-Y-, Oxazolyl-Y-, Isoxazolyl-Y-, Thiazolyl-Y-, Pyridyl-Y- oder Tetrazolyl-Y- darstellen, mit der Ausnahme, daß R⁴ und R⁵ nicht beide ausgewählt sind aus einem Wasserstoffatom und C₁-C₆-Alkyl und jeder heterocyclische Ring unsubstituiert oder durch C₁-C₆-Alkyl, C₁-C₆-Alkoxy, Cl, Br, F, I, NR⁷R⁷, CO₂R⁷, SO₂NHR⁷, SO₃H oder CONR⁷R⁷, OH, NO₂, W, SO₂W, SC₁-C₆-Alkyl, SO₂C₁-C₆-Alkyl, NR⁷COH, NR⁷COW oder NR⁷COC₁-C₆-Alkyl substituiert ist;
Y eine Einfachbindung, O, S oder ein C₁-C₆-Alkylrest, welcher geradkettig oder verzweigt oder gegebenenfalls durch Phenyl oder Benzyl substituiert ist, wobei jede der Arylgruppen unsubstituiert oder durch Halogen, NO₂, CF₃, C₁-C₆-Alkyl, C₁-C₆-Alkoxy, CN oder CO₂R⁷ substituiert ist;
R⁶ -Z-COOR⁸ oder -Z-CONR⁷R⁷ darstellt;
Z eine Einfachbindung, Vinyl, -CH₂-O-CH₂-, Methylen, gegebenenfalls substituiert durch C₁-C₆-Alkyl, eine oder zwei Benzylgruppen, Thienylmethyl oder Furylmethyl oder -C(O)NHCHR⁹-, darstellt, wobei R⁹ H, C₁-C₆-Alkyl, Phenyl, Benzyl, Thienylmethyl oder Furylmethyl darstellt;
W CₙF₂ₙ₊₁ darstellt, wobei n gleich 1-3 ist;
A -(CH₂)ₘ-, -CH=CH-, -O(CH₂)ₙ- oder -S(CH₂)ₙ- darstellt;
jeder Rest R⁷ unabhängig ein Wasserstoffatom, C₁-C₆-Alkyl oder (CH₂)ₘ-Phenyl darstellt, wobei m gleich 0 bis 4 ist; und
R⁸ ein Wasserstoffatom, C₁-C₆-Alkyl oder 2-Di(C₁-C₆-alkyl)-amino-2-oxoethyl darstellt; oder ein pharmazeutisch verträgliches Salz davon;
zur Herstellung eines Medikaments zur Behandlung von hämorrhagischem Schlaganfall.

2. Verwendung eines Angiotensin-II-Rezeptorantagonisten gemäß Anspruch 1, bei dem es sich um (E)-3-[2-n-Butyl-1-{(4-carboxyphenyl)methyl)-1H-imidazol-5-yl]-2-(2-thienyl)methyl-2-propensäure oder ein pharmazeutisch verträgliches Salz davon handelt.

3. Verwendung eines Angiotensin-II-Rezeptorantagonisten gemäß Anspruch 1, bei dem es sich um (E)-3-[2-n-Butyl-1-{(4-carboxyphenyl)methyl}-1H-imidazol-5-yl]-2-(2-thienyl)methyl-2-propensäure Methansulfonat handelt.

4. Verwendung eines Angiotensin-II-Rezeptorantagonisten gemäß Anspruch 1, bei dem es sich um (E)-3-[2-n-Butyl-1-{(4-carboxynaphth-1-yl)methyl}-1H-imidazol-5-yl]-2-(2-thienyl)methyl-2-Propensäure oder ein pharmazeutisch verträgliches Salz davon handelt.

5. Verwendung eines Angiotensin-II-Rezeptorantagonisten, bei dem es sich um 2-n-Butyl-4-chlor-1-[(2'-(1H-tetrazol-5-yl)biphenyl-4-yl)methyl]-5-(hydroxymethyl)-imidazol oder ein pharmazeutisch verträgliches Salz davon handelt; zur Herstellung eines Medikaments zur Behandlung von hämorrhagischem Schlaganfall.

6. Verwendung eines Angiotensin-II-Rezeptorantagonisten, bei dem es sich um 2-n-Propyl-4-pentafluorethyl-1-[(2'-(1H-tetrazol-5-yl)biphenyl-4-yl)methyl]imidazol-5-carbonsäure oder ein pharmazeutisch verträgliches Salz davon handelt; zur Herstellung eines Medikaments zur Behandlung von hämorrhagischem Schlaganfall.

7. Verwendung eines Angiotensin-II-Rezeptorantagonisten, bei dem es sich um 5,7-Dimethyl-2-ethyl-3-(2'-(tetrazol-5-yl)biphenyl-4-yl)methyl-3H-imidazo[4,5-b]pyridin oder ein pharmazeutisch verträgliches Salz davon handelt; zur Herstellung eines Medikaments zur Behandlung von hämorrhagischem Schlaganfall.

8. Verwendung eines Angiotensin-II-Rezeptorantagonisten, bei dem es sich um 2-Methyl-4-[(2'-(1H-tetrazol-5-yl)biphenyl-4-yl)methoxy]chinolin oder ein pharmazeutisch verträgliches Salz davon handelt; zur Herstellung eines Medikaments zur Behandlung von hämorrhagischem Schlaganfall.

## Revendications

1. Utilisation d'un antagoniste du récepteur d'angiotensine II, de formule : 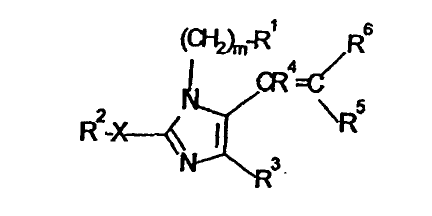 dans laquelle :
R¹ représente un groupe adamantyle, phényle, biphényle ou naphtyle, chaque groupe aryle étant non substitué ou substitué avec un à trois substituants choisis entre des substituants Cl, Br, F, I, alkyle en C₁ à C₆, nitro, A-CO₂R⁷, tétrazol-5-yl, alkoxy en C₁ à C₆, hydroxy, S(alkyle en C₁ à C₆), SO₂NHR⁷, NHSO₂R⁷, SO₃H, CONR⁷R⁷, CN, SO₂(alkyle en C₁ à C₆), PO(OR⁷)₂, NR⁷R⁷, NR⁷COH, NR⁷CO(alkyle en C₁ à C₆), NR⁷CON(R⁷)₂, NR⁷COW, W, SO₂W ;
m a une valeur de 0 à 4 ;
R² représente un groupe alkyle en C₂ à C₁₀, alcényle en C₃ à C₁₀, alcynyle en C₃ à C₁₀, cycloalkyle en C₃ à C₆, ou (CH₂)₀₋₈phényle non substitué ou substitué avec un à trois substituants choisis entre des substituants alkyle à C₁ à C₆, nitro, Cl, Br, F, I, hydroxy, alkoxy en C₁ à C₆, NR⁷R⁷, CO₂R⁷, CN, CONR⁷R⁷, W, tétrazol-5-yl, NR⁷CO(alkyle en C₁ à C₆), NR₇COW, S(alkyle en C₁ à C₆), SO₂W et SO₂(alkyle en C₁ à C₆) ;
X représente une liaison simple, S, un groupe NR⁷ ou O ;
R³ représente un atome d'hydrogène, un groupe Cl, Br, F, I, CHO, hydroxyméthyle, COOR⁷, CONR⁷R⁷, NO₂, W, CN, NR⁷R⁷, ou phényle ;
R⁴ et R⁵ représentent indépendamment un atome d'hydrogène, un groupe alkyle en C₁ à C₆, thiényl-Y-, furyl-Y-, pyrazolyl-Y-, imidazolyl-Y, pyrrolyl-Y-, triazolyl-Y-, oxazolyl-Y-, isoxazolyl-Y, thiazolyl-Y-, pyridyl-Y-, ou tétrazolyl-Y-, sauf que R⁴ et R⁵ ne sont pas choisis l'un et l'autre entre un atome d'hydrogène et un groupe alkyle en C₁ à C₆ et chaque noyau hétérocyclique est non substitué ou substitué avec un substituant alkyle en C₁ à C₆, alkoxy en C₁ à C₆, Cl, Br, F, I, NR⁷R⁷, CO₂R⁷, SO₂NHR⁷, SO₃H ou CONR⁷R⁷, OH, NO₂, W, SO₂W, S(alkyle en C₁ à C₆), SO₂(alkyle en C₁ à C₆), NR⁷COH, NR⁷COW, ou NR⁷CO(alkyle en C₁ à C₆).
Y représente une liaison simple, O, S ou un groupe alkyle en C₁ à C₆ qui est droit ou ramifié ou facultativement substitué avec un groupe phényle ou benzyle, chacun des groupes aryle étant non substitué ou substitué avec un substituant halogéno NO₂, CG₃, alkyle en C₁ à C₆, alkoxy en C₁ à C₆, CN ou CO₂R⁷ ;
R⁶ représente un groupe Z-COOR⁸ ou -Z-CONR⁷R⁷ ;
Z représente une liaison simple, un groupe vinyle, -CH₂-O-CH₂-, méthylène facultativement substitué avec un substituant alkyle en C₁ à C₆, un ou deux groupes benzyle, thiénylméthyle ou furylméthyle, ou -C(O)NHCHR⁹-, dans lequel R⁹ représente H, un groupe alkyle en C₁ à C₆, phényle, benzyle, thiényméthyle ou furylméthyle ;
W représente un groupe CₙF₂ₙ₊₁, dans lequel n a une valeur de 1 à 3 ;
A représente un groupe -(CH₂)ₘ-, -CH=CH-, -O(CH₂)ₙ ou -S(CH₂)n- ;
chaque groupe R⁷ représente indépendamment un atome d'hydrogène, un groupe alkyle en C₁ à C₆ ou (CH₂)ₘphényle, dans lequel m a une valeur de 0 à 4 ; et
R⁸ représente un atome d'hydrogène, un groupe alkyle en C₁ à C₆ ou 2-di(alkyle en C₁ à C₆)-amino-2-oxoéthyle ; ou d'un de ses sels pharmaceutiquement acceptables ;
dans la production d'un médicament destiné au traitement d'un ictus hémorragique.

2. Utilisation d'un antagoniste du récepteur d'angiotensine II suivant la revendication 1, qui est l'acide (E)-3-[2-n-butyl-1-{(4-carboxyphényl)méthyl}-1H-imidazol-5-yl]-2-(2-thiényl)méthyl-2-propénoique ou un de ses sels pharmaceutiquement acceptables.

3. Utilisation d'un antagoniste du récepteur d'angiotensine II suivant la revendication 1, qui est le méthanesulfonate d'acide (E)-3-[2-n-butyl-1-{(4-carboxyphényl)méthyl}-1H-imidazolyl-5-yl]-2-(2-thiényl)-méthyl-2-propénoïque.

4. Utilisation d'un antagoniste du récepteur d'angiotensine II suivant la revendication 1, qui est l'acide (E)-3-[2-n-butyl-1-{(4-carboxynapht-1-yl)méthyl}-1H-imidazol-5-yl]-2-(2-thiényl)méthyl-2-propénoïque ou un de ses sels pharmaceutiquement acceptables.

5. Utilisation d'un antagoniste du récepteur d'angiotensine II qui est le 2-n-butyl-4-chloro-1-[(2'-(1H-tétrazol-5-yl)biphényl-4-yl)méthyl]-5-(hydroxyméthyl)-imidazole ou un de ses sels pharmaceutiquement acceptables ;
dans la production d'un médicament destiné au traitement d'un ictus hémorragique.

6. Utilisation d'un antagoniste du récepteur d'angiotensine II qui est l'acide 2-n-propyl-4-pentafluoroéthyl-1-[(2'-(1H-tétrazol-5-yl)biphényle-4-yl)-méthyl]imidazole-5-carboxylique ou un de ses sels pharmaceutiquement acceptables ; dans la production d'un médicament destiné au traitement d'un ictus hémorragique.

7. Utilisation d'un antagoniste du récepteur d'angiotensine II qui est la 5,7-diméthyl-2-éthyl-3-(2'-(tétrazol-5-yl)biphényl-4-yl)méthyl-3H-imidazo[4,5-b]-pyridine ou un de ses sels pharmaceutiquement acceptables ; dans la production d'un médicament destiné au traitement d'un ictus hémorragique.

8. Utilisation d'un antagoniste du récepteur d'angiotensine II qui est la 2-méthyl-4-[(2'-(1H-tétrazol-5-yl)biphényl-4-yl)méthoxy]quinoléine ou un de ses sels pharmaceutiquement acceptables ;
dans la production d'un médicament destiné au traitement d'un ictus hémorragique.
